Europäisches Patentamt

European Patent Office

Office européen des brevets

(19)

(11) Numéro de publication : **0 198 878**
**B1**

(12) **FASCICULE DE BREVET EUROPÉEN**

(45) Date de publication du fascicule du brevet :
**19.07.89**

(21) Numéro de dépôt : **85905222.7**

(22) Date de dépôt : **24.10.85**

(86) Numéro de dépôt international :
**PCT/FR 85/00302**

(87) Numéro de publication internationale :
**WO/8602639 (09.05.86 Gazette 86/10)**

(51) Int. Cl.⁴ : **C 07 C155/06, A 61 K 31/325**

(54) **APPLICATION DU DIETHYLDITHIOCARBAMATE (DTC) A LA PREPARATION D'UN MEDICAMENT ET SON PROCEDE DE PREPARATION.**

(30) Priorité : **26.10.84 FR 8416393**

(43) Date de publication de la demande :
**29.10.86 Bulletin 86/44**

(45) Mention de la délivrance du brevet :
**19.07.89 Bulletin 89/29**

(84) Etats contractants désignés :
**AT BE CH DE FR GB IT LI NL SE**

(56) Documents cités :
**DE–A– 2 445 679
GB–A– 2 081 094
Fluka Katalog II, 1978, Fluka AG (CH), page 441, see no. 71480
Immunomodulatory Drugs and Modifiers of the Biological Response (Elsevier Biomedical Press, 1982) Chapter 9, G. Renoux et al.: "Immunopharmacaolgy of DTC in mice and men", pages 113-132
Médecine et Chirurgie Digestives, vol. 12, no. 7, 1983, G. Champault et al.: "L'Immuno-Stimulation en chirurgie viscérale essai thérapeutique contrôlé par diéthyl-dithio-carbamate de sodium (124 patients)", pages 537-543**

(73) Titulaire : **INSTITUT MERIEUX
17, rue Bourgelat
F-69002 Lyon (FR)**

(72) Inventeur : **BOUZINAC de la BASTIDE, René, Marie, Louis, Guy
43, allée des Tilleuls
F-69340 Francheville (FR)**
Inventeur : **CHARBONNIER, Claude, Jean
8, rue de Charavey
F-69290 St-Genis-les-Ollières (FR)**
Inventeur : **MUSSET, Mircea
21, avenue du Général Leclerc
F-69160 Tassin-La-Demi-Lune (FR)**

(74) Mandataire : **Lemoine, Michel et al
Cabinet Michel Lemoine et Bernasconi 13 Boulevard des Batignolles
F-75008 Paris (FR)**

Journal de Pharmacologie (Paris), vol. 13, Suppl. I, G. Renoux: "Immunopharmacolgie et pharmacologie du diéthydithiocarbamate (DTC)", pages 95-134

Biological Response Modifiers in Human Oncology and Immunology, Thomas Klein et al. (Plenum publishing corporation), 1983, G. Renoux et al.: "Sodium diethyldithiocarbamate (Imuthiol) and Cancer", pages 223-239

Clin. Immunology and Immunophathology, vol. 15, no. 1, 1980, G. Renoux et al.: "The effects of sodium diethyldithiocarbamate, Azathioprine, cyclophosphamide or hydrocortisone acetate administered alone or in association for 4 weeks on the immune response of BALB/c mice", pages 23-32

Journal of Immunopharmacology, vol. 2, no. 1, 1980, G. Renoux et al.: "Induction of differetiation of human null cells into T lymphocytes under the influence of serum of mice with sodium diethyldithiocarbamate", pages 49-59

Thymus, vol. 2, no. 3, 1980, G. Renoux et al.: "Administration of DTC gives evidence of a role of the thymus in the control and regulation of factors inducing thymocyte differentiation in the mouse", pages 139-146

Int. J. Immunopharmac., vol. 4, no. 4, 1982, M. Roumaintzeff et al.: "DTC, an immunophotentiator specific for T cells", page 286

Journal de Pharmacologie (Paris), vol. 15, no. 4, 1984, G. Renoux: "Pharmacological properties of Imuthiol", pages 486-487

# EP 0 198 878 B1

## Description

La présente invention a trait à une application du diéthyldithiocarbamate (DTC) à la préparation d'un médicament antiviral ainsi qu'à un procédé d'utilisation de ce médicament pour la prévention ou le traitement de maladies virales, y compris le SIDA.

Elle a également trait à un procédé de préparation de diéthyldithiocarbamate (DTC) pratiquement dépourvu de constituants toxiques et ayant un degré élevé d'activité sur le système immunitaire et à un médicament à base de ce DTC purifié, ainsi qu'à l'application de ce DTC purifié à la préparation d'un tel médicament ainsi qu'à un procédé d'utilisation de ce médicament pour la prévention ou le traitement de maladies virales, y compris le SIDA, et pour la prévention ou le traitement, y compris au long cours, d'autres maladies, notamment chez les nourrissons et enfants, les personnes âgées, et les immuno-déprimés.

On a déjà décrit, dans le brevet français 2 269 960 et dans le brevet US-4 148 885, à titre de nouveau médicament immuno-stimulant, des thiols minéraux, parmi lesquels le diéthyldithiocarbamate de sodium (DTC).

D'autres références relatives au DTC comprennent, par exemple :

— Gérard Renoux et al. Immunopharmacology of DTC in mice and men, Immunomodulatory Drugs and Modifiers of the Biological Response, Elsevier Biomedical Press pp. 113-132, 1982.

— Gérard Renoux et al. Sodium diethyldithiocarbamate (Imuthiol) and cancer, Biological Response Modifiers in Human Oncology and Immunology, Ed. by Thomas Klein, Steven Specter, Herman Friedman and Andor Szentivanyl, Plenum Publishing Corporation, pp. 223-239, 1983.

— Gérard Renoux. Recent Advances in Immunopharmacology and Immunotherapy, Recent Advances in Tumor Immunology : Oncogenes to Tumor Antigens, Anacapri, May 13-15, 1984.

En fait, le diéthyldithiocarbamate qui était disponible pour ces travaux était loin d'être un produit parfaitement purifié et comportait une teneur importante en impuretés mal connues.

Des essais de toxicité sur souris, rats et chiens effectués avec une préparation davantage purifiée et lyophilisée de DTC ont également montré une réduction de la toxicité chez les animaux — Voir Gérard Renoux, Immunopharmacologie et Pharmacologie du Diéthyldithiocarbamate (DTC), J. Pharmacol. (Paris), 1982, 13, suppl. I 95-134.

La présente invention se propose donc de fournir un procédé de préparation d'un médicament à base de DTC à partir du DTC disponible dans le commerce, qui soit libre d'effets toxiques, à un degré non encore atteint chez les patients, et qui soit susceptible d'être utilisé, aux doses efficaces, notamment pour des traitements au long cours, chez les patients les plus sensibles à la toxicité y compris les enfants, les nourrissons et les personnes âgées.

Un tel médicament, tout en ayant un large spectre d'activité, est particulièrement utile pour le traitement et la prévention d'affections virales liées à des dérèglements immunitaires chez l'enfant ou le nourrisson, et les personnes âgées.

Un autre objectif de l'invention est d'appliquer le DTC à la préparation d'un médicament actif dans la prévention et le traitement des maladies virales, y compris du syndrome immuno-déficitaire acquis (SIDA), y compris des manifestations associées (ARC) et pré-SIDA.

Un autre objectif de l'invention est de fournir un procédé de préparation d'un médicament à base de DTC, à partir du DTC disponible dans le commerce, qui soit libre d'effets toxiques, à un degré non encore atteint, chez les patients souffrant de maladies virales, y compris chez les immuno-déprimés et chez les malades atteints du SIDA, y compris de ses manifestations associées (ARC) et pré-SIDA.

Un autre objectif de l'invention est de permettre l'obtention d'un effet anti-viral, y compris in vitro.

Un autre objectif de l'invention est de fournir un procédé de purification des diéthyldithiocarbamates, notamment de sodium, à partir du DTC disponible dans le commerce qui supprime pratiquement les problèmes de toxicité en mettant en œuvre une technique de purification à l'eau dans des conditions telles que la tendance de cette matière à se dégrader spontanément dans les milieux aqueux ne constitue pas un obstacle.

La présente invention a pour objet un nouveau médicament anti-viral comprenant une préparation purifiée de diéthyldithiocarbamate (DTC) caractérisé en ce que le DTC a été purifié par dissolution dans un milieu aqueux, ledit DTC étant conditionné pour l'administration.

De façon préférée, le DTC commercial est dissous dans une quantité suffisante d'eau, sous agitation, par exemple pendant un quart d'heure, après quoi la solution est d'abord clarifiée par passage sur membrane de porosité 150 à 250 microns, de préférence en verre fritté, puis sur une membrane stérilisante de l'ordre de 0,2 micron. De préférence, les étapes en phase aqueuse sont effectuées en moins de 24 heures. On effectue ensuite une lyophilisation à une température inférieure à 40 °C. La substance traitée par ce procédé a été trouvée libre des impuretés gênantes ou toxiques précédentes et est fortement active en tant qu'immunomodulateur.

Pour appliquer cette substance à la préparation d'un médicament à administer par voie orale, on réhydrate ensuite la substance et on effectue ensuite une granulation humide de la substance au degré de granulométrie recherché. Le médicament peut alors être conditionné pour l'administration par voie orale.

Pour appliquer cette substance à la préparation d'un médicament à administrer par voie intra-

3

veineuse, la lyophilisation est effectuée directement dans les flacons prévus pour l'application du traitement.

Le médicament obtenu selon ce procédé peut être administré de préférence par voie orale ou par voie intraveineuse.

Pour l'administration par voie orale, le médicament est de préférence conditionné sous forme de gélules gastroprotégées. On constate de façon surprenante que ces gélules restent stables dans leur présentation et dans leur coloration aux températures habituelles de conservation alors que les gélules qui avaient été préparées avec des préparations de diéthyldithiocarbamate de sodium antérieurement connues voyaient leur couleur et leur aspect se modifier et se dégrader avec le temps.

Pour l'administration par voie intraveineuse, la préparation selon l'invention est de préférence lyophilisée et prévue pour être dissoute dans un solvant spécial.

Les dosages par voie orale sont de préférence de l'ordre de 125 mg pour l'adulte et 50 mg pour l'enfant. Les dosages en flacon lyophilisé sont de préférence de l'ordre de 125 mg et 500 mg. Les doses sont avantageusement prévues pour une administration hebdomadaire.

L'effet sur l'organisme récepteur n'est pas dose-dépendant. Cependant, les doses préférées de traitement sont, par voie orale, de l'ordre de 10 mg par kg par semaine pour l'enfant et l'adulte et, par voie intraveineuse, de l'ordre de 5 mg par kg par semaine. De préférence, la dose hebdomadaire est administrée en une seule fois.

Le médicament ainsi préparé peut être utilisé pour le rétablissement de l'activité immunitaire, notamment chez les nourrissons, les enfants et les personnes âgées, chez les immunodéprimés.

On a découvert de façon particulièrement surprenante que cette nouvelle préparation purifiée de DTC était active dans le traitement du SIDA et de ses manifestations associées telles qu'ARC et pré-SIDA, alors que les propriétés du DTC étaient considérées comme particulièrement contre-indiquées dans cette affection. On a alors découvert que le DTC possède une activité anti-virale inattendue in vitro sur le virus HTLV-III/LAV.

La présente invention a donc également pour objet l'application du DTC, de préférence mais non exclusivement de la substance purifiée de DTC selon le procédé décrit ci-dessus, à la préparation d'un médicament anti-viral ainsi qu'à un médicament anti-viral préparé à partir du DTC purifié selon le procédé décrit ci-dessus, de préférence conditionné sous forme de gélules gastroprotégées ou de préparation lyophilisée à administration parentérale, notamment intraveineuse.

Parmi les maladies virales traitées grâce à l'invention, on peut citer celles dues aux virus suivants :

— Retroviridae, notamment HTLV-III/LAV.
— Herpesviridae, notamment Alphaherpesviridae (y compris HSV 1 et HSV 2).
— Papovaviridae, y compris papillomavirus (notamment papillomatose laryngée de l'enfant).
— Poxviridae.
— Picornaviridae.
— Virus de l'hépatite B (HBV).

D'autres avantages et caractéristiques de l'invention apparaîtront à la lecture de la description suivante, faite à titre d'exemple non limitatif.

#### Exemple 1 — Purification de la préparation commerciale de diéthyldithiocarbamate de sodium

La substance de départ est une préparation de diéthyldithiocarbamate de sodium en provenance de la société CLEVENOT, Darmstadt, République Fédérale Allemande. Cette préparation présente les caractéristiques physiques suivantes : Couleur : Blanchâtre, tirant sur le jaune ou le rose. Degré d'hydratation : 23 à 25 %.

On introduit 13 kg de cette préparation dans un volume de 50 litres d'eau pure dépyrogénée, en l'absence de toute substance métallique tant au niveau du récipient qu'au niveau de l'agitateur. La dissolution est complète, à température ambiante, après environ un quart d'heure.

On effectue ensuite une filtration clarifiante sur membrane en verre fritté de porosité 200 microns environ, toujours à température ambiante, ceci pendant une durée de 30 minutes environ.

La solution clarifiée est ensuite soumise à une filtration sur membrane stérilisante 0,2 micron.

Cette filtration s'effectue environ en 30 à 60 minutes.

On effectue ensuite une lyophilisation sous vide avec une température finale n'excédant pas 40 °C. Cette lyophilisation dure environ 48 heures.

On obtient ainsi une substance lyophilisée d'un poids de 10 kg.

On réhydrate cette masse et on effectue une granulation humide de cette masse pour obtenir un degré de granulométrie de l'ordre de 800 microns.

#### Exemple 2 — Conditionnement

La substance est ensuite conditionnée sous forme de gélules de 50 et 125 mg.

Pour les préparations par voie intraveineuse, on effectue simplement l'étape précitée de lyophilisation directement dans les flacons prévus pour l'application.

On prépare, pour la dissolution, un liquide de dissolution dont la composition est la suivante :

| | | |
|---|---|---|
| Phosphate monosodique | $NaH_2PO_4 2H_2O$ | 1,00 g |
| Phosphate disodique | $Na_2HPO_4 , 12H_2O$ | 3,70 g |
| Chlorure de sodium | NaCl | 8,00 g |
| Edétate de sodium | $C_{10}H_{14}N_2Na_2O_8 , 2H_2O$ | 0,5 g |
| Eau p.p.i. | QSP 1000 ml | |
| NaOH QS pH = 7,0 | | |

## Exemple 3 — Activité anti-virale in vitro

Les essais ont été effectués sur deux types de lymphocytes, à savoir normaux HPLB et lignée H9, par infection in vitro par le virus HTLV III/LAV et incubation simultanée en présence de dithiocarbamate de sodium à une concentration de $10^{-6}$ et $10^{-9}$ g/ml.

Les résultats sont les suivants, exprimés en pourcentages par rapport aux quantités déterminées dans la préparation infectée témoin :

| | HPBL | H9 |
|---|---|---|
| Reverse transcriptase | 30 % | 70 % |
| Protéine virale p 15 | 50 % | 55 % |
| Protéine virale p 24 | 60 % | 50 % |

Ils montrent une inhibition d'environ 50 % de la reverse transcriptase et de l'expression des protéines virales p.5 et p. 24.

## Exemple 4 — Traitement de l'ARC (complexe relatif àu SIDA)

Un essai pilote sans contrôle a été réalisé chez des patients mâles complètement informés sous administration hebdomadaire de 5-10 mg/kg de poids corporel pendant 3 à 6 mois avec les résultats suivants.

$T_4$ + PBL (lymphocytes du sang périphérique)
chez des patients ARC

| Patients | avant | après | durée du traitement |
|---|---|---|---|
| 1 | 10 % (102) | 42 % (672) | 5 mois |
| 2 | 24 % (494) | 21 % (504) | 6 mois |
| 3 | 43 % (2400) | 50 % (1275) | 6 mois |
| 4 | 14 % (157) | 22 % (330) | 3 mois |
| 5 | 26 % (468) | 33 % (267) | 4 mois |
| 6 | 34 % (697) | 33 % (429) | 6 mois |

### $T_8 + PBL$ chez des patients ARC

| Patients | avant | après | durée du traitement |
|---|---|---|---|
| 1 | 17 % (173) | 35 % (560) | 5 mois |
| 2 | 21 % (432) | 30 % (720) | 6 mois |
| 3 | 18 % (1008) | 23 % (586) | 6 mois |
| 4 | 8 % (90) | 10 % (150) | 3 mois |
| 5 | 33 % (594) | 22 % (178) | 4 mois |
| 6 | 32 % (656) | 17 % (221) | 6 mois |

### E-ROSETTES chez des patients ARC

| Patients | avant | après | durée du traitement |
|---|---|---|---|
| 1 | 33 % (337) | 70 % (1120) | 5 mois |
| 3 | 39 % (2184) | 64 % (1632) | 6 mois |
| 4 | 34 % (382) | 73 % (1095) | 3 mois |
| 5 | 55 % (900) | 66 % (534) | 4 mois |

### DCH chez des patients ARC
### DCH score (hypersensibilité retardée)

| Patients | avant | après | durée du traitement |
|---|---|---|---|
| 1 | 0 | 8,5 | 5 mois |
| 2 | 7,5 | 17,5 | 6 mois |
| 3 | 0 | 7 | 6 mois |
| 4 | 2,5 | 2,5 | 3 mois |
| 5 | 0 | 23 | 6 mois |

Ces résultats montrent l'effet positif du médicament selon l'invention, montré par la surveillance des paramètres immunologiques et l'amélioration clinique relevée chez tous les patients.

En outre, on pense que le médicament selon l'invention est utile dans le traitement des désordres immunitaires (y compris les déficiences de lymphocytes T).

### Exemple 5 — Traitement d'herpès récidivant chez deux enfants

Sujet n° 1 — DN 8/11/72 présentant des poussées d'herpès cornéen depuis 1978. Taux élevé d'anticorps herpès sans déficit sérique. Posologie 50 mg/semaine depuis mars 1983. Aucune récidive n'est survenue.

Sujet n° 2 — DN 16/4/78 présentant des herpès cutanés récidivants et streptococcie cutanée ayant mal réagi à l'antibiothérapie. Le bilan immunitaire montrait une hypoIgG à 400 mg et une diminution du CH 50 (complément hémolyse 50 %) à 40. Posologie 10 mg/kg/semaine depuis septembre 1984. Aucune poussée d'herpès n'est survenue depuis.

**Revendications**

1. Procédé de préparation de diéthyldithiocarbamate (DTC), notamment de sodium, caractérisé par une toxicité réduite, dans lequel on dissout du DTC commercial dans de l'eau apyrogène, on filtre la solution et on récupère le DTC de l'eau.

2. Procédé selon la revendication 1, caractérisé en ce que l'on effectue la récupération par lyophilisation à une température finale qui ne dépasse pas 40 °C.

3. Procédé selon l'une des revendications 1 et 2, dans lequel la filtration comprend les étapes consistant à clarifier la solution à travers une membrane de porosité environ 100 microns à 250 microns puis à la filtrer sur membrane stérilisante à environ 0,2 micron.

4. Procédé selon la revendication 3, caractérisé en ce que la durée totale des étapes en phase gazeuse ne dépasse pas 24 heures.

5. Procédé de préparation d'un médicament pour la prévention et le traitement de maladies virales, y compris le SIDA et ses manifestations associées (ARC) et pré-SIDA, l'herpès, caractérisé en ce que l'on utilise le diéthyldithiocarbamate (DTC) et qu'on le conditionne pour l'administration.

6. Procédé selon la revendication 5, caractérisé en ce que le DTC est du diéthyldithiocarbamate de sodium.

7. Procédé selon l'une quelconque des revendications 5 et 6, caractérisé en ce que le DTC a été purifié par dissolution dans un milieu aqueux.

8. Procédé selon la revendication 7, caractérisé en ce que le DTC commercial est dissous dans une quantité suffisante d'eau, sous agitation, après quoi la solution est d'abord clarifiée par passage sur membrane de porosité 150 à 250 microns, puis passée sur une membrane stérilisante de l'ordre de 0,2 micron.

9. Procédé selon la revendication 8, caractérisé en ce que les étapes en phase aqueuse sont effectuées en moins de 24 heures.

10. Procédé selon l'une des revendications 5 à 9, caractérisé en ce qu'on conditionne le DTC sous forme de gélule gastroprotégée.

11. Procédé selon l'une quelconque des revendications 5 à 9, caractérisé en ce qu'on conditionne le DTC sous forme lyophilisée adaptée à une injection parentérale, notamment intraveineuse.

12. Médicament pour la prévention et le traitement des maladies virales, caractérisé en ce qu'il comprend une préparation de diméthyldithiocarbamate (DTC), notamment de sodium, purifiée par dissolution dans un milieu aqueux, le DTC étant conditionné pour l'administration.

13. Médicament selon la revendication 12, caractérisé en ce qu'il est conditionné sous doses pour une administration hebdomadaire, notamment, pour la voie orale de 125 mg pour l'adulte et 50 mg pour l'enfant et, pour la voie parentérale de 125 mg et 500 mg.

**Claims**

1. A process for preparing diethyldithiocarbamate (DTC), in particular of sodium, characterized by a low toxicity, in which commercial DTC is dissolved in apyrogenic water, the solution is filtered and the DTC is recovered from the water.

2. A process according to claim 1, characterized in that the recovery is effected by lyophilization at a final temperature which is not higher than 40 °C.

3. A process according to one of the claims 1 and 2, wherein the filtration comprises the steps consisting of clarifying the solution through a membrane having a porosity of about 100 microns to 250 microns, then filtering it on a sterilizing membrane of about 0.2 micron.

4. A process according to claim 3, characterized in that the total duration of the steps in an aqueous phase does not exceed 24 hours.

5. A process for preparing a drug for the prevention and treatment of viral diseases, including AIDS and its associated manifestations (ARC) and pre-AIDS, and herpes, characterized in that diethyldithiocarbamate (DTC) is used and put into condition for administration.

6. A process according to claim 5, characterized in that the DTC is sodium diethyldithiocarbamate.

7. A process according to any one of the claims 5 and 6, characterized in that the DTC has been purified by dissolving it in an aqueous medium.

8. A process according to claim 7, charaterized in that the commercial DTC is dissolved in a sufficient quantity of water while stirring, whereafter the solution is first of all clarified by passage through a membrane having a porosity of 150 to 250 microns, then passed through a sterilizing membrane of on the order of 0.2 micron.

7

9. A process according to claim 8, characterized in that the steps in an aqueous phase are carried out within less than 24 hours.

10. A process according to any one of the claims 5 to 9, characterized in that the DTC is put into the form of a gastroprotected gelule.

11. A process according to any one of the claims 5 to 9, characterized in that the DTC is put into a lyophilized form adapted for a parenteral and in particular intravenous injection.

12. A drug for the prevention and treatment of viral diseases, characterized in that it comprises a preparation of diethyldithiocarbamate (DTC), in particular of sodium, purified by dissolving it in an aqueous medium, the DTC being put into condition for administration.

13. A drug according to claim 12, characterized in that it is put into condition for doses for a weekly administration, in particular, by the oral route, of 125 mg for the adult and 50 mg for the child and, by the parenteral route, of 125 mg and 500 mg.


**Patentansprüche**

1. Verfahren zur Herstellung von Diethyldithiocarbamat (DTC), insbesondere des Natriums, gekennzeichnet durch eine verminderte Toxizität, in welchem man kommerzielles DTC in nicht pyrogenem Wasser löst, die Lösung filtriert und das DTC aus dem Wasser gewinnt.

2. Verfahren nach Anspruch 1, dadurch gekennzeichnet, daß die Gewinnung durch Gefriertrocknung bei einer Endtemperatur, die 40 °C nicht überschreitet, erfolgt.

3. Verfahren nach einem der Ansprüche 1 und 2, in welchem die Filtration als Schritte das Klären der Lösung durch eine Membran mit einer Porosität von etwa 100 μm bis 250 μm und danach das Filtrieren durch eine sterilisierende Membran von etwa 0,2 μm emfaßt.

4. Verfahren nach Anspruch 3, dadurch gekennzeichnet, daß die Gesamtdauer der Schritte in wäßriger Phase 24 h nicht überschreitet.

5. Verfahren zur Herstellung eines Medikaments zur Vorbeugung und Behandlung von Viruskrankheiten, unter Einschluß von AIDS und damit verbundene Manifestierungen (ARC) und prä-AIDS sowie Herpes, dadurch gekennzeichnet, daß man Diethyldithiocarbamat (DTC) verwendet und es für die Verabreichung konditioniert.

6. Verfahren nach Anspruch 5, dadurch gekennzeichnet, daß das DTC Natriumdiethyldithiocarbamat ist.

7. Verfahren nach einem der Ansprüche 5 und 6, dadurch gekennzeichnet, daß das DTC durch Lösen in einem wäßrigen Medium gereinigt worden ist.

8. Verfahren nach Anspruch 7, dadurch gekennzeichnet, daß kommerzielles DTC unter Rühren in einer ausreichenden Menge Wasser gelöst wird, wonach die Lösung zuerst durch Passieren durch eine Membran der Porosität 150 bis 250 μm geklärt und danach durch eine sterilisierende Membran der Größenordnung 0,2 μm geführt wird.

9. Verfahren nach Anspruch 8, dadurch gekennzeichnet, daß die Schritte in der wäßrigen Phase in weniger als 24 h bewirkt werden.

10. Verfahren nach einem der Ansprüche 5 bis 9, dadurch gekennzeichnet, daß man das DTC in Form einer gegen Magensaft beständigen Gelkapsel bringt.

11. Verfahren nach einem der Ansprüche 5 bis 9, dadurch gekennzeichnet, daß man das DTC in eine gefriergetrocknete Form bringt, die für eine parenterale, insbesondere intravenöse, Injektion geeignet ist.

12. Medikament zur Vorbeugung und Behandlung von viralen Krankheiten, dadurch gekennzeichnet, daß es eine Zubereitung von Diethyldithiocarbamat (DTC), insbesondere des Natriums, gereinigt durch Auflösen in einem wäßrigen Medium, umfaßt, wobei das DTC für die Verabreichung konditioniert ist.

13. Medikament nach Anspruch 12, gekennzeichnet, durch eine Konfektionierung in Dosen für eine wöchentliche Verabreichung, insbesondere 125 mg pro Erwachsenem und 50 mg pro Kind für den oralen Weg und 125 mg bzw. 500 mg für den parenteralen Weg.